**Europäisches Patentamt**

(19) **European Patent Office**

**Office européen des brevets**

(11) Publication number: **0 478 272 A2**

## (12) EUROPEAN PATENT APPLICATION

(21) Application number: **91308673.2**

(22) Date of filing: **24.09.91**

(51) Int. Cl.⁵: **C07K 15/14, C12P 21/08, G01N 33/577, G01N 33/68, A61K 39/395, A61K 37/02**

(30) Priority: **24.09.90 US 586685**

(43) Date of publication of application:
**01.04.92 Bulletin 92/14**

(84) Designated Contracting States:
**AT BE CH DE DK ES FR GB GR IT LI LU NL SE**

(71) Applicant: **BECTON, DICKINSON & COMPANY**
**One Becton Drive**
**Franklin Lakes New Jersey 07417-1880 (US)**

(72) Inventor: **Phillips, Joseph H.**
**15 Pine Avenue**
**San Carlos, California 94070 (US)**
Inventor: **McKinney, Lisa**
**2618-4 Abbey Drive**
**Fort Wayne, Indiana 46835 (US)**
Inventor: **Lanier, Lewis L.**
**1528 Frontero Avenue**
**Los Altos, California 94024 (US)**

(74) Representative: **Ruffles, Graham Keith et al**
**MARKS & CLERK 57-60 Lincoln's Inn Fields**
**London WC2A 3LS (GB)**

(54) **Gp98 cell adhesion molecule and integrin complex containing it.**

(57) A purified integrin glycoprotein complex (cell adhesion molecule) is provided composed of β4, α6, and a novel 98 kD subunit (gp98). Also provided are antibodies to the cell adhesion molecule, additional polypeptides capable of binding to antibodies specific for the molecule, and nucleic acid sequences encoding the molecule.

EP 0 478 272 A2

## BACKGROUND OF THE INVENTION

### Field of the Invention

This invention relates to an intgrin complex imposed of β4, α6, and gp98 subunits and specifically to a gp98 kD cell-surface glycoprotein expressed on colon and other cells, antibodies directed to the glycoprotein, nucleic acid sequences encoding the glycoprotein, and diagnostic tests for assaying the presence of the glycoprotein.

### Description of the Background

Specific interactions between lymphocytes and other cells are an essential feature of the immune system. Lymphocytes must be able to attach to other cells so that they may participate in the inflammation response and interact with antigen-presenting cells. Lymphocytes interact with other cells and with the basal lamina through a variety of surface protein adhesion molecules present on the lymphocyte and its binding target.

Integrins are a type of cell surface adhesion molecule that are composed at $\alpha$ and $\beta$ glycoprotein subunits. These heterodimers are involved in adhesion with other cell surface ligands and extracellular matrix proteins (Albelda et al., Faseb J. (1990), 4:2868). It has been assumed that integrins are all heterodimers and that they do not contain additional subunits. One such integrin complex that has been identified on colon carcinomas is the β4,α6 complex (Hemler et al., J. Biol. Chem. (1989), 264:6529).

NK cells are a population of lymphocytes identified by the phenotype $CD_3^-$, $CD_{56}^+$ that mediate MHC-unrestricted cytotoxicity against certain malignant and virus-infected cells, as well as produce numerous cytokines upon immune stimulation. NK cells constitutively express intermediate affinity receptors for IL-2 and are able to kill a broad spectrum of tumor cell targets after activation by IL-2.

Although the receptor used for NK-cell recognition of tumors has not been identified, the CD18/CD11a integrin complex, also referred to as β2/LFA-1 integrin, is involved in the cytotoxic process against certain tumors. The CD11a/CD18 complex has previously been shown to specifically bind to two different cellular ligands found on target cells, namely ICAM-1, also known as CD54 (Marlin et al., Cell (1987) 51:813-819), and ICAM-2 (Staunton et al., Nature (1989) 339:61-64). Antibodies specific for CD18 have been shown to block killing of some target cells bearing ICAM-1 or ICAM-2 incubated with IL-2-stimulated NK cells.

The identification of interacting pairs of proteins that mediate cell-cell recognition in the immune system enables one to modulate the immune response. For example, modulation of the immune response may be achieved by preparing antibodies directed to individual members of an interacting pair of cell-surface proteins. Additionally, modulation of the immune response may be achieved by preparing polypeptides homologous to one member of a specifically interacting pair of surface proteins so as to competitively block the interaction between the surface protein binding pair.

Assays for the detection of cells bearing a specific cell interaction protein are also of interest. Such assays may be used to determine the presence and quantity of a specific type of cell characterized by the expression of a particular cell interaction molecule.

## SUMMARY OF INVENTION

The present invention provides a novel, substantially purified, cell-surface glycoprotein complex composed of β4, α6, and gp98 subunits in non-covalent association. The invention also provides for purified gp98, fragments of gp98, antibodies specific for gp98, and nucleotide sequences encoding gp98. The subject invention also provides means of modulating aspects of immune response controlled by cell to cell interactions involving the β4,α6,gp98 complex.

## DESCRIPTION OF SPECIFIC EMBODIMENTS

A new cell glycoprotein complex, referred to hereafter as β4,α6,gp98, has been found on colon adenocarcinoma cell lines and normal colon epithelial tissues. While β4 and α6 have been identified previously, the specific complex β4,α6,gp98 is novel, as is the gp98 glycoprotein itself. The present invention provides purified β4,α6,gp98 complex, purified gp98, nucleic acid sequences encoding gp98, antibodies specific to the β4,α6,gp98 complex, and polypeptide fragments of gp98. The invention also provides monoclonal antibodies capable of reacting with the β4,α6,gp98 complex and thereby preventing functional interactions between cells bearing this complex and lymphocytes.

The β4,α6,gp98 complex was discovered by preparing murine monoclonal antibodies to a human adenocarcinoma cell line, Colo-205. The resulting monoclonal antibodies were screened for their ability to inhibit killing of Colo-205 adenocarcinoma cells by interleukin 2 stimulated NK cells. A number of different monoclonal antibodies with the desired properties were found, including L279 (IgG3 isotype), L280 (IgG1 isotype) and L281 (IgM isotype). For the purposes of binding to the β4,α6,gp98 complex on colon carcinoma and inhibiting NK cell-mediated cytotoxicity against Colo-205, all three antibodies work efficiently. One of these hybridomas, L280, is deposited with the American Type Culture Collection, 12301 Parklawn Drive, Rockville, Maryland, 20852, USA (ATCC deposit number           ).

In this invention, the novel gp98 glycoprotein is defined as a glycoprotein having the properties as described herein as isolated from a mammalian species, humans being a particularly preferred species, as well as various derivatives thereof, also described herein. Glycosylated gp98 has a molecular weight (Mr) of about 98 Kd as determined by sodium dodecyl sulfate polyacrylamide gel electrophoresis (SDS-PAGE). When the N-linked sugars are removed from gp98, for example by EndoF treatment as described in Lanier et al., J. Exp. Med. (1988) 67:1572, the deglycosylated protein is found to have an SDS-PAGE Mr of about 55 Kd. Hereinafter gp98 used without indicating whether the protein is glycosylated or unglycosylated will refer to the unglycosylated protein, except when it is indicated that gp98 is isolated from cells naturally producing gp98. When it is indicated that gp98 is isolated from natural sources of gp98, it will be understood that glycosylated gp98 is intended. By the term "glycosylated" it is intended that carbohydrates are attached to gp98 (and derivatives thereof) in a manner substantially the same as the glycosylation of gp98 isolated from natural sources. However, partial deglycosylation or addition of other sugar units can occur while still retaining the glycosylated gp98 designation.

The compound gp98, both glycosylated and unglycosylated, or polypeptide fragments thereof, may be used for producing antibodies, either monoclonal or polyclonal, specific to glycosylated and/or unglycosylated gp98. By polypeptide fragments of gp98 is meant polypeptides differing in length from natural gp98 and containing five or more, preferably 10 or more, more preferably 20 or more, amino acids from gp98 in the same primary order as found in gp98 as obtained from a natural source, up to the total amino acid sequence of the molecule. Such molecules are not required to be prepared by fragmentation of an entire gp98 molecule, although such fragments can be prepared in that manner, but can also be prepared by synthetic techniques, either chemically or by genetic engineering. Fragments, as well as gp98 itself, can be derivatized to increase stability, if desired. Polypeptide molecules having substantially the same amino acid sequence as gp98 but possessing minor amino acid substitutions that do not substantially affect the ability of the gp98 polypeptide derivatives to interact with gp98-specific molecules or receptors for gp98 on cells (such as NK cells) are within the definition of gp98 itself. Molecules that are gp98-specific include polypeptides such as antibodies that are specific for the gp98 polypeptide containing the naturally occurring gp98 amino acid sequence. By "specific binding polypeptide" is intended polypeptides that bind with gp98 and its derivatives and which have a measurably higher binding affinity for the target polypeptide, i.e., gp98 and polypeptide derivatives of gp98, than for other polypeptides tested for binding. Higher affinity by a factor of 10 is preferred, more preferably a factor of 100. Binding affinity for antibodies refers to a single binding event (i.e., monovalent binding of an antibody molecule). Specific binding by antibodies also means that binding takes place at the normal binding site of the antibody (at the end of the arms in the variable region) rather than by non-specific binding at other locations. Binding affinity of at least $10^7$ is preferred, especially at least $10^8$ and most preferably at least $10^9$.

As discussed above, minor amino acid variations from the natural amino acid sequence of gp98 are contemplated as being encompassed by the term gp98; in particular, conservative amino acid replacements are contemplated. Conservative replacements are those that take place within a family of amino acids that are related in their side chains. Genetically encoded amino acids are generally divided into four families: (1) acidic = aspartate, glutamate; (2) basic = lysine, arginine, histidine; (3) non-polar = alanine, valine, leucine, isoleucine, proline, phenylalanine, methionine, tryptophan; and (4) uncharged polar = glycine, asparagine, glutamine, cystine, serine, threonine, tyrosine. Phenylalanine, tryptophan, and tyrosine are sometimes classified jointly as aromatic amino acids. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding properties of the resulting molecule, especially if the replacement does not involve an amino acid at a binding site involved in the interaction of sp98 or its derivative with a lymphocyte, especially an NK cell. Whether an amino acid change results in a functional peptide can readily be determined by assaying the specific binding properties of the gp98 polypeptide derivative.

Antibodies specific for gp98 are produced by immunizing an appropriate vertebrate host, e.g., rabbit or rodent, with cells expressing gp98, purified gp98, extracts of cells containing gp98, or polypeptide derivatives of gp98, by themselves or in conjunction with a conventional adjuvant. Usually, two or more immunizations will be involved, and blood or spleen will be harvested a few days after the last injection. For polyclonal antisera, the immunoglobulins can be precipitated, isolated and purified from serum by a variety of standard techniques, including affinity purification using gp98 attached to a solid surface, such as a gel or beads in an affinity column. For monoclonal antibodies, the splenocytes normally will be fused with an immortalized lymphocyte, e.g., a myeloma cell line, under selective conditions for hybridoma formation. The hybridomas can then be cloned under limiting dilution conditions and their supernatants screened for antibodies having the desired specificity.

Techniques for producing antibodies are well-known in the literature and are exemplified by the publication Antibodies: A Laboratory Manual (1988) eds. Harlow and Lane, Cold Spring Harbor Laboratories Press, and U.S. Patent Nos. 4,381,292, 4,451,570, and 4,618,577.

For example, mAbs were generated against Colo-205 and selected for the ability to inhibit cytotoxicity mediated by IL-2-activeted NK cells. Several mAbs, designated L279 (IgG3,κ isotype), L280 (IgG,κ), and L281 (IgM,κ), were identified that efficiently blocked NK-cell-mediated cytotoxicity against Colo-205. L280 mAb inhibited the cytolytic activity of a polyclonal population of peripheral blood NK cells cultured overnight in IL-2, as well as the function of IL-2-dependent NK clones. Monoclonal antibody against other membrane proteins expressed on Colo-205 including β4 (3E1 mAb), α6 (GoH3 MAb), and β1 (A11B2 mAb), as well as RGD peptide, failed to inhibit NK-cell-mediated killing of Colo-205.

The antigen specificity of L279, L280 and L281 mAb were distinct from anti-β1 and anti-α6 since they did not react with peripheral blood leukocytes, platelets, or any hematopoietic tumor cell line examined (including Jurkat, U937, KG1a, JY, and K562). However, examination of normal tissue revealed that these mAbs specifically reacted with epithelial cells in colon and breast. Additional details on the production and properties of these antibodies are set forth in the examples that follow. These examples are not limiting of the invention but rather give specific instructions on how preferred aspects of the invention including mAbs can be practiced.

The β4,α6,gp98 complex can be readily purified from cells naturally producing the polypeptide and from cells genetically modified to produce derivatives thereof by affinity chromatography using a monoclonal antibody specific for the β4,α6,gp98 complex, such as the L280 antibody previously described. Once the β4,α6,gp98 complex is obtained, further antibodies can be prepared using the initial complex or using gp98 isolated from the complex, and the further antibodies can be utilized to purify future batches of gp98. In addition to the use of antibody affinity chromatography, gp98 and polypeptide derivatives thereof can be purified by a variety of other widely known protein purification techniques (either alone or in combination) including immunoprecipitation, gel filtration, ion exchange chromatography, chromatofocusing, isoelectric focusing, selective precipitation, electrophoresis, and the like. Fractions isolated during purification procedures can be analyzed for the presence of β4,α6,gp98 complex or polypeptide derivatives of gp98 by immunoassays employing gp98-specific antibodies or gp98-specific bioassays. Detailed examples are provided below.

For example, Colo-205 cells were surface labeled with $^{125}$I and detergent solubilized, and glycoproteins were isolated by lentil lectin affinity chromatography. A glycoprotein complex composed of at least 5 proteins (having molecular weights of 170, -150, 135, 118, and 98 kD) was immunoprecipitated using the L280, L279, and L281 mAbs. This complex was similar to the β4,α6 integrin that is preferentially expressed on colon carcinomas. As noted previously for the β4,α6 complex, the relative labeling intensity of the different chains varied between experiments, and in some experiments an additional band of about 190 kD was observed. Published studies have indicated that β4 exists in isoforms of several sizes, based on proteolytic cleavage of the cytoplasmic segment. Direct comparative analysis indicated that while two mAb against β4 (3E1 and 439-9B) and two mAb against α6 (GoH3 and J1-B5) immunoprecipitated the 170, 150, 135, and 118 kD proteins, only L280 immunoprecipitated an additional gp98 subunit. That α6 and β4 are components of the complex was demonstrated by the ability to affinity purify the complex using L280 mAb-Sepharose, elute the proteins, and then re-immunoprecipitate the eluted proteins with anti-β4, anti-α6, and L280 mAb. While L280 mAb re-immunoprecipitated all of the original components of the complex, anti-β4 and anti-α6 only immunoprecipitated the higher molecular weight structures and not gp98. This indicates that anti-β4 and anti-α6 are unable to react with a β4,α6 complex containing gp98 and that binding of these mAb may dissociate gp98 from the complex. The gp98 subunit is in non-covalent association with β4,α6, as demonstrated by SDS-PAGE analysis under non-reducing and reducing conditions. The α6 subunit migrated below the diagonal after reduction on these two-dimensional gels, due to the cleavage of a small disulfide linked component of about 25 kD, whereas the β4 polypeptides and gp98 migrated slightly above the diagonal as a consequence of intrachain disulfide bonds.

Since a proteolytic fragment of β4 has previously been observed to migiate at 85 kD, further studies were undertaken to provide evidence that gp98 is a distinct polypeptide. Removal of N-linked oligosaccharides with endo-F reduced the mobility of the gp98 subunit in a manner different from that of known subunits. Additionally, the L280 mAb that reacts with the β4,α6,gp98 complex has been shown not to react with the binary β4,α6 complex. Data showing these results are set forth in the examples that follow. Although it appears from this data that L280 mAb reacts with the gp98 subunit, this has not been directly confirmed since this mAb fails to react in Western blot analysis. Peptide mapping experiments have also further indicated that gp98 is distinct from the β4 glycoprotein.

Possession of purified gp98 and/or antibodies specific for gp98 permits the isolation of nucleotide sequences encoding gp98. Techniques for isolating gene sequences encoding a specific protein when one skilled in the art possesses the purified protein and/or antibodies specific to the protein of interest are well known in the literature. See, e.g., Molecular Cloning: A Laboratory Manual, 2nd Ed. (1989) by Sambrook et al., Cold Spring Harbor Laboratories Press.

Isolation of nucleotide sequences encoding gp98 involves creation of either a genomic library prepared from cells encoding gp98 or preparation of a cDNA library from RNA isolated from cells expressing gp98. It will generally be preferable to create a cDNA library for isolation of gp98 coding nucleotide sequences so as to avoid any possible problems arising from attempts to determine intron/exon borders. Genetic libraries can be made in either eukaryotic or prokaryotic host cells. Widely available cloning vectors such as plasmids, cosmids, phage, YACs and the like can be used to generate genetic libraries suitable for the isolation of nucleotide sequences encoding gp98 or portions thereof.

Useful methods for screening genetic libraries for the presence of gp98 encoding nucleotide sequences include the preparation of oligonucleotide probes based on the N-terminus amino acid sequenceinformation from purified gp98 or purified internal fragments of purified gp98. By employing the standard triplet genetic code, oligonucleotide sequences of about 17 base pairs or longer can be prepared by conventional in vitro synthesis techniques so as to correspond to portions of gp98 for which the amino acid sequence has been determined by N-terminus analysis (or by such analysis of proteolytic fragments). The resultant nucleic acid sequences can be subsequently labeled with radionuclides, enzymes, biotin, fluorescers, or the like, and used as probes for screening genetic libraries.

Additional methods of interest for isolating gp98 encoding nucleic acid sequences include screening genetic libraries for the expression of gp98 or fragments thereof by means of gp98-specific antibodies, either polyclonal or monoclonal.

Nucleotide sequences encoding gp98 can be obtained from recombinant DNA molecules recovered from gp98-specific genetic library isolates. The nucleotide sequence encoding gp98 can be obtained by sequencing the non-vector nucleotide sequences of these recombinant molecules. Nucleotide sequence information can be obtained by employing widely used DNA sequencing protocols, such as Maxim and Gilbert sequencing, dideoxy nucleotide sequencing, and the like. Examples of suitable nucleotide sequencing protocols can be found in Berger and Kimmel, Methods in Enzymology Vol. 52, Guide to Molecular Cloning Techniques, (1987) Academic Press. Nucleotide sequence information from several recombinant DNA isolates, including isolates from both cDNA and genomic libraries, may be combined so as to provide the entire amino acid coding sequence of gp98, as well as the nucleotide sequences of introns within the gp98 gene, upstream nucleotide sequences, and downstream nucleotide sequences.

Nucleotide sequences obtained from sequencing gp98 specific genetic library isolates are subjected to analysis in order to identify regions of interest in the gp98 gene. These regions of interest include open reading frames, introns, promoter sequences, termination sequences, and the like. Analysis of nucleotide sequence information is preferably performed by computer. Software suitable for analyzing nucleotide sequences for regions of interest is commercially available and includes, for example, DNASIS™ (LKB). It is also of interest to use amino acid sequence information obtained from the N-terminus sequencing of purified gp98 when analyzing gp98 nucleotide sequence information so as to improve the accuracy of the nucleotide sequence analysis.

Isolated nucleotide sequences encoding gp98 can be used to produce purified gp98 (either glycosylated or unglycosylated) or fragments-thereof by either recombinant DNA methodology or by in vitro polypeptide synthesis techniques. By "purified" and "isolated" is meant, when referring to a polypeptide or nucleotide sequence, that the indicated molecule is present in the substantial absence of other biological macromolecules of the same type. However, it is recognized that with glycoproteins, some variation in the sugar portion of the molecules in collection of molecules that has been isolated or purified. The term "purified" as used herein preferably means at least 95% by weight, more preferably at least 99% by weight, and most preferably at least 99.8% by weight, of biological macromolecules of the same type present (but water, buffers, and other small molecules, especially molecules having a molecular weight of less than 1000, can be present).

A significant advantage of producing gp98 by recombinant DNA techniques is that equivalent quantities of gp98 can be produced by using less starting material and other reagents than would be required for isolating gp98 from a natural source. Producing gp98 by recombinant techniques also permits gp98 to be isolated in the absence of some molecules normally present in cells that naturally produce gp98. It is also apparent that recombinant DNA techniques can be used to produce gp98 polypeptide derivatives that are not found in nature, such as the variations described above.

The gp98 of the invention and polypeptide derivatives of gp98 can be expressed by recombinant techniques when a DNA sequence encoding the relevant molecule is functionally inserted into a vector. By "functionally inserted" is meant in proper reading frame and orientation, as is well understood by those skilled in the art. When producing a genetic construction containing a complete gp98 reading frame, the preferred starting material is a cDNA library isolate encoding gp98 rather than a genomic library isolate. Typically, the gp98 gene will be inserted downstream from a promoter and will be followed by a stop codon, although production as a hybrid protein followed by cleavage may be used, if desired. In general, host-cell-specific sequences improving the production yield of gp98 and gp98 polypeptide derivatives will be used and appropriate control sequences will be added to the expression vector, such as enhancer sequences, polyadenylation sequences, and ribosome binding sites.

Expression of gp98 and polypeptide derivatives thereof can be enhanced by including multiple copies of the gp98 gene (or derivative thereof) in a transformed host, by selecting a vector known to reproduce in the host, thereby producing large quantities of protein from exogenous inserted DNA (such as pUC8, ptac12, or pIN-III-ompA1, 2, or 3), or by any other known means of enhancing peptide expression.

In addition to the above general procedures which can be used for preparing recombinant DNA molecules and transformed unicellular organisms in accordance with the practices of this invention, other known techniques and modifications thereof can be used in carrying out the practice of the invention. In particular, techniques relating to genetic engineering have recently undergone explosive growth and development. Many recent U.S. patents disclose plasmids, genetically engineering microorganisms, and methods of conducting genetic engineering which can be used in the practice of the present invention. For example, U.S. Patent 4,273,875 discloses a plasmid and a process of isolating the same. U.S. Patent 4,304,863 discloses a process for producing bacteria by genetic engineering in which a hybrid plasmid is constructed and used to transform a bacterial host. U.S. Patent 4,419,450 discloses a plasmid useful as a cloning vehicle in recombinant DNA work. U.S. Patent 4,362,867 discloses recombinant cDNA construction methods and hybrid nucleotides produced thereby which are useful in cloning processes. U.S. Patent 4,403,036 discloses genetic reagents for generating plasmids containing multiple copies of DNA segments. U.S. Patent 4,363,877 discloses recombinant DNA transfer vectors. U.S. Patent 4,356,270 discloses a recombinant DNA cloning vehicle and is a particularly useful disclosure for those with limited experience in the area of genetic engineering since if defines many of the terms used in genetic engineering and the basic processes used therein. U.S. Patent 4,336,336 discloses a fused gene and a method of making the same. U.S. Patent 4,349,629 discloses plasmid vectors and the production and use thereof. U.S. Patent 4,332,901 discloses a cloning vector useful in recombinant DNA. Although some of these patents are directed to the production of a particular gene product that is not within the scope of the present invention, the procedures described therein can easily be modified to the practice of the invention described in this specification by those skilled in the art of genetic engineering.

A wide variety of hosts may be employed for expression of the subject polypeptides, both prokaryotic and eukaryotic. Useful hosts include bacteria, such as E. coli, yeast, filamentous fungus, immortalized mammalian cells, such as various mouse lines, monkey lines, chinese hamster ovary lines, human lines, or the like. For the most part, the mammalian lines will be immortalized by transformation to a neoplastic state, where the cells may be isolated from a neoplastic host, or wild-type cells may be isolated from a neoplastic host, or wild-type cells may be transformed with oncogenes, tumor causing viruses, or the like. Eukaryotic cells are preferred, and mammalian cell lines are particularly preferred hosts, for the production of glycosylated gp98 and glycosylated derivatives thereof because of the glycosylation systems present in these cells but generally absent in procaryotic cells.

The nucleotide sequence information obtained from isolation of the nucleotide sequence encoding gp98 can be used to produce polypeptides by mans of in vitro synthesis. Such synthesis is conveniently achieved through the use of a commercially available polypeptide synthesis machine, such as a model 431A peptide synthesizer available from Applied Biosystems. Such equipment provides ready access to the peptides of the invention, either by direct synthesis or by synthesis of a series of fragments that can be coupled using other known techniques.

It is of interest to detect $\beta4,\alpha6$,gp98-complex- or gp98-containing cells when they appear in regions of the body where they do not occur in healthy individuals, e.g., during the metastasis of colorectal tumors. The detection of free, i.e., not bound to a cell surface, $\beta4,\alpha6$,gp98 complex or gp98 or naturally occurring derivatives thereof is also of interest because the appearance of free gp98 or $\beta4,\alpha6$,gp98 complex may be associated with pathological states, such as cancer. Antibodies to gp98 or $\beta4,\alpha6$,gp98 complex (glycosylated and unglycosylated) and its fragments find use in immunoassays for the detection of gp98 or $\beta4,\alpha6$,gp98 complex. These antibodies and polypeptides can be employed in assay procedures that are well described in the literature. These assays include ELISA, RIA, western blotting, immunohistochemical staining, and the like. In general, appropriate assays will employ immunoglobulin molecules or portions of immunoglobulin molecules, either labeled or unlabeled, that are specific for $\beta4,\alpha6$,gp98 complex or gp98. Assays of interest also can include in the assay medium predetermined ammounts of gp98, polypeptide derivatives thereof, or polypeptide-containing fragments of gp98 added for the purpose of acting as a competitive inhibitor of a specific binding reaction when gp98 or a complex containing gp98 is the analyte.

The subject invention specifically contemplates modulating the immune response in mammals by administering effective amounts of gp98 or β4,α6,gp98 complex-specific antibodies (including for example antibody fragments or antibody conjugates), gp98 (glycosylated and unglycosylated), a polypeptide containing a portion of the gp98 sequence, or polypeptide containing fragments of gp98 when the gp98 is isolated from cells naturally producing gp98. By "modulating" is intended either increasing or decreasing the intensity of a given immune response for a given administered molecule. Whether an increase or decrease will occur can be determined experimentally using, e.g., the techniques described in the examples that follow. The polypeptides and antibodies described herein will have an effect on interaction between lymphocytes, especially NK cells, and β4,α6,gp98-complex- or gp98-bearing cells. Experimental data set forth below demonstrate that lysis of gp98- or β4,α6,-gp98-complex-bearing cells by NK cell is inhibited by the addition of anti-β4,α6,gp98-complex antibodies. Thus, administration of gp98-based polypeptides and gp98 or β4,α6,gp98-complex-specific immunoglobulins, with a physiologically acceptable carrier if necessary, will reduce the immune response. Of particular interest is the interruption of the inflammation response in autoimmune diseases.

Additional uses for the compounds and complexes of the invention include as targeting molecules for in vivo imaging and therapy, in which the targeting molecule is part of a complex that also includes an active component, such as a radioactive element or toxin. The targeting molecule (either a gp98-based molecule or an antibody or other specificbinding compound) allows selective distribution upon administration of the complex so that the active component can exert its effect at the desired location. Such uses are well known with other targeting molecules, and the knowledge of the art can be applied directly to use of compounds and complexes of the invention as targeting molecules in commination with known active components.

The availability of purified gp98 compositions of the invention and antibodies thereto make a number of compositions derivable from such components available for the first time. For example, chimeric antibodies can be prepared using variable regions from mouse antibodies as described herein and constant regions from human antibodies. Synthetic variable regions can also be made based on amino acid sequences of antibodies of the invention and tested for binding to gp98 molecules.

The invention now being generally described, the same will be better understood by reference to the following detailed examples which are provided for purposes of illustration only and are not to be considered limiting of the invention unless so specified.

EXAMPLES

Production of β4,α6,qp98 Complex-Specific Monoclonal Antibodies

Balb/c mice were immunized with Colo-205 colon adenocarcinoma tumor cells (publicly available from the American Type Culture Collection, Rockville, Maryland, USA, under the designation ATCC CCL 222). Hybridomas secreting monoclonal antibodies were produced by fusing lymphoid cells from the spleen of the immunized mice with cells from plasmocytoma cell line Sp2/0 using conventional fusion techniques. Several alternative mouse plastocytoma lines are publicly available from the ATCC (e.g., P3/Ns1/1-Ag4-1 is available as ATCC TIB 18).

Supernatants from the hybridomas were screened for the ability to block IL-2-activated NK cell killing of $^{51}$Cr-radiolabeled Colo-205 carcinoma cells. The method for IL-2 activation of NK cells and the cytotoxicity assays using Colo-205 targets have been described previously (Phillips and Lanier (1988) J. Exp. Med. 164:814). To screen for blocking antibodies, hybridoma supernatants (typically 100 μl supernatant) were added to the $^{51}$Cr-labeled Colo-205 cells for 30 min., and then IL-2-activated NK effector cells were added at an effector to target ratio of 25:1. After 4 hours incubation at 37°C, radioisotope released into the supernatant was measured using a gamma counter. Amount of radioisotype from the Colo-205 cells is considered to be proportional to the degree of tumor cell death. Hybridoma supernatants that prevented target cell killing by IL-2-activated NK cells by at least 25% were cloned and characterized. Three hybridomas of particular interest are designated L279, L280, and L281 in this specification.

Immunoprecipitation of β4,α6,pg98 complex

Colo-205 and HT-29 (ATCC, Rockville, MD) cells were surface labeled with $^{125}$I(Amersham, Arlington Heights, IL) and were solubilized in Tris-buffered saline (TBS, 50 mM Tris, 150 mM NaCl, pH 8.0) containing 1% NP-40, 20 Kallikrein inhibitor units/ml aprotinin (Sigma, St. Louis, MO) and 1 mM PMSF (Sigma). This technique is described in detail in Lanier et al., J. Exp. Med. (1987) 165:1076. Cell lysates were passed through a 0.2 ml (bed volume) column of Dowex 1x8-400 (chloride form, Sigma) equilibrated in TBS to remove unbound $^{125}$I and were precleared three times with 10 mg Pansorbin (Calbiochem-Behring, San Diego, CA) coated with saturating amounts of rabbit anti-mouse Ig serum (Lanier et al., J. Exp. Med. (1987) 165:1076). Lysates were passed through lentil lectin-Sepharose 4B (Pharmacia, Piscataway, NJ) equilibrated in loading buffer (10 mM Tris, 150 mM NaCl, 0.25% NP-40, 0.05% NaN$_3$ , pH 7.4), and glycoproteins were eluted in loading buffer containing 300 mM 1-0-methyl-α-D-glucopyranoside (Sigma). Antigens were immunoprecipitated using Pansorbin coated with saturating amounts of rabbit anti-mouse Ig and mAb (Lanier et al., J. Ex. Med. (1987) 165:1076). Monoclonal antibodies used were anti-β4 mAb (3E1, purchased from Telios Pharmaceuticals, San Diego, CA, and 439-9B, provided by Dr. S. Kennel and described in Falcioni et al., Cancer Research (1988) 48:816) and anti-α6 (GoH3, provided by Dr. A. Sonnenberg and described in Hemler, et al., J. Biol. Chem. (1989) 264:6529 and J1B5, provided by Dr. C. Damsky). Immunoprecipitates were washed five times in TBS containing 1% NP-40. Samples were analyzed by SDS-PAGE under reducing conditions, or by two-dimensional diagonal SDS-PAGE (1st dimension non-reducing, 2nd dimension reducing) using 7.5% acrylamide gels (Lanier et al., J. Exp. Med. (1987) 165:1076).

A glycoprotein complex composed of at least 5 proteins (170, 150, 135, 118, and 98 kD) was immunoprecipitated using the L280 antibody. This complex was similar to the β4,α6 integrin that is preferentially expressed on colon carcinomas (Hemler, et al., J. Biol. Chem. (1989) 264:6529). As noted previously for the β4,α6 complex (Hemler, et al., J. Biol. Chem. (1989) 264:6529), the relative labeling intensity of the different chains varied between experiments, and in some experiments an additional band of 190 kD was observed. Recent studies have indicated that β4 exists in isoforms of several sizes, based on proteolytic cleavage of the cytoplasmic segment (Hogervorst et al., EMBO J. (1990) 9:765). Direct comparative analysis indicated that while two mAb against β4 (3E1 and 439-9B) and two mAb against α6 (GoH3 and J1B5) immunoprecipitated the 170, 150, 135, and 118 kD proteins, only L280 immunoprecipitated on additional gp98 subunit.

### L280 mAb co-immunoprecipitates the β4 and α6 integrins

Prior studies have demonstrated the existence of an integrin complex on colon carcinomas, composed of β4 and α6 subunits (Hemler et al., J. Biol. Chem. (1989) 264:6529). The gp98 molecule was not observed in this complex; however, the tri-molecular complex immuno-precipitated by L280 mAb appeared to contain β4 and α6 subunits. This was confirmed by biochemical analysis. $^{125}$I-labeled lentil-lectin-purified Colo-205 glyco-proteins (described above) were incubated for 2 hrs with 25 μl Sepharose 4B coupled by CNBr to L280 mAb (about 3 mg Ig per ml of beads coupled as described in Lanier et al., J. Immunol. (1986) 137:2286). Beads were washed five times in 1% NP-40 TBS, and antigen was eluted in 50 μl of 50 mM diethylamine (pH 12) and immediately neutralized with 50 μl 1 M Tris pH 7.6. Eluted antigen was diluted to 1 ml in cold 1% NP-40 TBS containing 10 mg/ml bovine serum albumin and 1 mM PMSF (Sigma). This purified antigen was re-immunoprecipitated with control mAb, anti-β4 (439-9B),anti-α6 (J1B5), or L280 (as described above). Samples were analyzed by SDS-PAGE under reducing conditions using 7.5% acrylamide gels (Lanier et al., J. Exp. Med. (1987) 165:1076). These studies prove that β4 and α6 are components of the novel β4,α6,gp98 complex immunoprecipitated by L280 mAb.

### Deglycosylation of β4,α6,gp98 complex and evidence that gp98 is a unique subunit

For removal of N-linked oligosaccharides, L280 mAb immunoprecipitates were treated with 65 U/ml endo-F (New England Nuclear, Boston, MA) for 24 h at 37°C in the presence of 1 mM PMSF and 50 mM EDTA (Lanier et al., J. Exp. Med. (1988), 167:1572). Samples were analyzed by SDS-PAGE under reducing conditions, using 7.5% acrylamide gels (Lanier et al., J. Exp. Med. (1987) 165:1076).

Removal of N-linked oligosaccharides with endo-F reduced the mobility of the β4 and α6 subunits by 5-10 kD consistent with prior studies (Kennel et al., J. Biol. Chem. (1989) 264:15515); however, gp98 was diminished to 55 kD. This high degree of N-linked glycosylation clearly distinguishes gp98 from β4 and α6. HT-29, another colon carcinoma cell line that expresses the β4,α6 complex, failed to react with the L280 mAb. Since L280 mAb does not react with HT-29 or platelets, this excludes the possibility that this antibody is directed against β4 or α6, respectively.

### Effect of anti-β4,α6,gp98 antibodies on NK cell activity

Monoclonal antibodies generated against Colo-205 adenocarcinoma cells were selected on the basis of the ability to inhibit IL-2 activated NK cell-mediated killing of Colo-205 (see above). Three monoclonal antibodies, designated L279 (IgG3,κ isotype), L280 (IgG1,κ) and L281 (IgM,κ) were identified that efficiently blocked NK-cell-mediated cytotoxicity against Colo-205. These mAb inhibited the cytolytic activity of a polyclonal population of peripheral blood NK cells cultured overnight in IL-2, as well as the function of IL-2-dependent NK clones. Monoclonal Ab concentration-dependent inhibition of cytotoxicity was demonstrated using purified L280 mAb. Monoclonal Ab against other membrane proteins expressed on Colo-205 including β4 (3E1 mAb), α6 (GoH3 mAb), and β1 (AllB2 mAb, generously provided by Dr. C. Damsky), as well as RGD peptide and human laminin (purchased from Telios Pharmaceuticals, San Diego, CA), failed to inhibit NK-cell-mediated killing of Colo-205.

Purification of β4,α6,gp98

A scheme for purifying β4, α6, gp98 complex is provided based on procedures described in Practical Immunology 3rd Edition, Hudson and Hay, (1989) Blackwell Scientific Publications. Colo-205 cells are solubilized in a non-ionic detergent such as Triton X-100™. A first affinity column of Sepharose CL-4B coupled to monoclonal antibody L-280 and a second affinity column of Sepharose CL-4B coupled to a control, e.g., non-β4,α6,gp98-specific antibody (either polyclonal or monoclonal), are prepared. Each column is equilibrated with a buffer of 10 mM Tris-HCl, pH 8.2, containing 0.5% sodium deoxycholate. Non-specific binding sites in the columns are saturated by running through 10 mM Tris-HCl buffer containing 1 mg/ml BSA (bovine serum albumin), then washing through with 10 mM Tris-HCl 0.5% deoxycholate buffer. The solubilized cells are applied to the control column, and the effluent is collected and applied to the column containing the L280 monoclonal antibodies. The column is washed with 3 volumes of the Tris-HCl-deoxycholate buffer. The bound material is eluted by treating the monoclonal antibody column with 0.05 M diethylamine-HCl buffer pH 11.5 containing 0.5% w/v deoxycholate. The eluate is titrated back to pH 8.5 with solid glycine and dialyzed against Tris-HCl-deoxycholate buffer. If a further purification cycle is required, the sample is applied to the specific antibody column again (following re-equilibration). The presence of β4,α6,gp98 complex in the eluate is measured by an immunoassay employing one of the antibodies described herein, such as L280.

All publications and patent applications mentioned in this specification are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

## Claims

1. A substantially purified polypeptide selected from the group consisting of gp98 and fragments thereof having an amino acid sequence comprising at least 5 adjoining amino acids of the gp98 amino acid sequence.

2. A protein complex containing the gp98 polypeptide according to Claim 1, wherein said polypeptide specifically binds to monoclonal antibody L280.

3. A protein complex according to Claim 2, wherein said complex comprises β4, α6, and gp98.

4. A monoclonal antibody capable of specifically binding glycosylated gp98, unglycosylated gp98, or a β4, α6, gp98 complex but not a β4, α6 complex.

5. An immunoassay for the detection of gp98 or a β4, α6, gp98 complex comprising the steps of:
   removing a sample for analysis from a mammal, wherein said sample is suspected of containing gp98 or a β4, α6, gp98 protein complex;
   adding to said sample a composition comprising an antibody capable of forming a specific-binding complex with said gp98 or said β4, α6, gp98 protein complex; and
   detecting formation of said specific-binding complex.

6. An immunoassay according to Claim 5, said immunoassay further comprising a step of adding a composition comprising a polypeptide having an amino acid sequence comprising at least 5 adjoining amino acids of the gp98 amino acid sequence, wherein said polypeptide is selected to bind specifically with said antibody, and measuring competitive binding of said polypeptide with said antibody in the presence of said sample.

7. An immunoassay according to Claim 5, wherein said gp98 is soluble.

8. A method for reducing an immune response in an animal, comprising:
   administering to said animal an effective amount of (1) an antibody capable of specifically binding gp98 or β4, α6, gp98 complex but not a β4, α6 complex or (2) soluble gp98 or a polypeptide fragment of gp98 capable of inhibiting cytotoxicity mediated by IL-2-activated NK cells.

9. The method Claim 8, wherein said immune response is an inflammation.

10. The method of Claim 8, wherein in said immune response is an autoimmune response.